(19) Europäisches Patentamt / European Patent Office / Office européen des brevets

(11) **EP 4 265 216 A1**

(12) **EUROPEAN PATENT APPLICATION**

(43) Date of publication:
**25.10.2023 Bulletin 2023/43**

(21) Application number: **22169326.0**

(22) Date of filing: **21.04.2022**

(51) International Patent Classification (IPC):
**A61B 34/30** (2016.01)          **A61B 34/00** (2016.01)
**B25J 7/00** (2006.01)          **B25J 17/02** (2006.01)

(52) Cooperative Patent Classification (CPC):
**A61B 34/30; A61B 50/00; A61B 90/04;**
**B25J 19/0054; B25J 19/0075; B25J 19/06;**
A61B 2050/0014; A61B 2090/0436

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Microsure B.V.**
**5692 EA Son (NL)**

(72) Inventors:
• **Van Gerven, Lars**
  **5382KC Vinkel (NL)**
• **Van Loon, Sebastiaan**
  **5653EX Eindhoven (NL)**

• **Kursten, Jacobus Marinus Andreas**
  **5725CN Asten (NL)**
• **Bosscher, Kasper**
  **5616GX Eindhoven (NL)**
• **Chatrou, Martijn Lambertus Laurentius**
  **5692VN Son en Breugel (NL)**
• **Van der Walle, Dirk**
  **686BB Oosterbeek (NL)**
• **Denasi, Alper**
  **5623BB Eindhoven (NL)**
• **Bezemer, Paul**
  **5702TC Helmond (NL)**

(74) Representative: **DTS Patent- und Rechtsanwälte
Schnekenbühl und Partner mbB
Brienner Straße 1
80333 München (DE)**

(54) **ROBOT ARM FOR USE IN SURGERY, MICROSURGERY OR SUPER-MICROSURGERY**

(57)     The present invention relates to a robot arm (100) for use in surgery, microsurgery or super-microsurgery, said robot arm (100) comprising:

a first, proximal arm element (102) having a first arm length (L1), the proximal arm element (102) being connected to a first, proximal joint (104) having a first roll (106) and a first pitch (108), and a second, intermediate joint (110) having a second roll (112) and a second pitch (114);

a second, intermediate arm element (116) having a second arm length (L2), the intermediate arm element (116) being connected to the intermediate joint (110) and a third, distal joint (118) having a third roll (120) and a third pitch (122), and a third, distal arm element (124) having a third arm length (L3), the third arm element (124) being connected to the distal joint (118),

wherein the ratios between the first to second to third arm element lengths (L1, L2, L3) are:

$$(1.00 +\!\!- 10\%) : \{ (1.00 \text{ to } 1.05) +\!\!- 10\% \} : (0.60 +\!\!- 10\%).$$

Fig. 1

**Description**

The present invention belongs to the technical field of robots for use in surgery, microsurgery or super-microsurgery procedures.

In particular, the present invention relates to a robot arm for use in surgery, microsurgery or super-microsurgery procedures.

For instance, the robot arm can be used in performing anastomoses

In surgery procedures, in particular microsurgery or super-microsurgery procedures, robots are used for precise surgical handlings on the submillimeter scale.

In particular, the use of robots facilitates accurate movements with micrometer precision without substantial tremor, thereby superseding the limitations of human hand manipulation.

However, it is necessary that these robots provide the operator (e.g., a surgeon) with sufficient dexterity, so as to be able to perform complex movements in the microsurgical workspace and also avoid the risk of collision with other objects during manipulation.

Further, it is important that parts of the robot, in particular the robot arm, do not interfere with and thus obstruct the microscopic view of the operator.

This is particularly relevant considering the environmental volume constraints of the surgical, in particular microsurgical and super-microsurgical workspace.

Robots for use in surgical, microsurgical or super-microsurgical procedures are known in the art.

For instance, EP2731535A1 discloses a microsurgical robotic device for providing robotic assistance during tasks that require long-term user concentration and high precision. One or multiple master-slave units are coupled to a central microscope-based suspension structure. The microsurgical robotic devices pay attention to motion scaling and tremor filtration in a 6 Degrees-of-Freedom (DOF) master-slave setup with force feedback. An extra DOF is included to actuate a 1-DOF instrument tip.

A drawback of this known robotic device is that link lengths are still suboptimal.

As a consequence, it is not possible to provide the operator with sufficient dexterity that is required to perform a wide range of microsurgical techniques (e.g., anastomoses).

A further drawback is that parts of the robot, in particular the robot arm, may interfere with and thus obstruct the operator's view on the surgical site during surgery.

In view of the above, it is an object of the present invention to provide an improvement for a robot arm for use in surgery, microsurgery or super-microsurgery, inter alia in that a robot arm is capable of providing the operator with enhanced precision and dexterity, at the same time maintaining a high level of accuracy and coping with volume constraints of the surgical, in particular microsurgical and super-microsurgical workspace.

The above object is obtained by the provision of a robot arm according to claim 1. Accordingly, a robot arm for use in surgery, microsurgery or super-microsurgery is provided, said robot arm comprising:

a first, proximal arm element having a first arm length, the proximal arm element being connected to a first, proximal joint having a first roll and a first pitch, and a second, intermediate joint having a second roll and a second pitch;

a second, intermediate arm element having a second arm length, the intermediate arm element being connected to the intermediate joint and a third, distal joint having a third roll and a third pitch, and

a third, distal arm element having a third arm length, the third arm element being connected to the distal joint,

wherein the ratios between the first to second to third arm element lengths are:
(1.00 +- 10%) :{ (1.00 to 1.05) A distal end of proximal arm element is connected to a second, intermediate joint.

In particular, the intermediate joint has a second roll and a second pitch.

The robot arm further includes a second, intermediate arm element having a second arm element length.

A proximal end of the intermediate arm element is connected to the intermediate joint. A distal end of the intermediate arm element is connected to a third, distal joint.

In particular, the distal joint has a third roll and a third pitch.

The robot arm further includes a third, distal arm element having a third arm element length.

A proximal end of the distal arm element is connected to the distal joint.

In particular, the ratios between the first to second to third arm elements lengths are:

$$(1.00 +- 10\%) : \{(1.00 \text{ to } 1.05) +- 10\%\} : (0.60 +- 10\%).$$

The invention is based on the basic idea that, by providing a robot arm for use in surgery, in particular microsurgery and super-microsurgery, having the above specified ratio's between arm elements lengths, it is possible to provide the operator (e.g., a surgeon) with enhanced dexterity and also prevent the risk of collision with other objects or even the patient during manipulation. Further, the operator may be provided with a better vision of the surgical site, without significant obstructions. Still further, the dimensions of the robot arm (and in particular of the end effector) can be reduced, such that less mass needs to be moved during manipulation. Further, the improved kinematics allows operating the robot arm with less power and at lower temperatures. The disclosed ratios have been found by experiment and experience and have shown to be very effective to achieve inter alia the above-mentioned objects.

Optionally, a sterile drape can be used to cover the robot arm for maintaining sterility conditions in the course of the surgical procedure.

Preferably, the ratios between the first to second to third arm elements lengths can be:

$$(1.00 +- 3\% \text{ to } 5\%) : \{(1.00 \text{ to } 1.05) +- 3 \text{ to } 5\%\} : (0.60 +- 3\% \text{ to } 5\%).$$

Advantageously, the first arm element length can be between 225 and 275 mm.

Advantageously, the second arm element length can be between 231,75 and 283,25 mm.

Advantageously, the third arm element length can be between 135 and 165 mm. Preferably, the first arm element length is 250 mm.

Preferably, the second arm element length is 257.5 mm.

Preferably, the third arm element length is 150 mm.

In particular, the robot arm may be configured to have six degrees of freedom (DOF) at its tip.

Preferably, the six degrees of freedom may include three translational degrees of freedom and three rotational degrees of freedom.

Accordingly, it is possible to perform surgical, in particular microsurgical and super-microsurgical operations (e.g., anastomoses) with greater accuracy and enhanced maneuverability when compared to the prior art.

Precision and accuracy of movements as well as maneuverability of the robot arm are further improved by the specific features and ranges of motion of the proximal, intermediate and distal joints.

In particular, the proximal joint may advantageously include the following ranges of motion:

first roll: -41° to +41° with respect to the first roll rotation axis, and/or

first pitch: -23° to +44° with respect to the first pitch rotation axis.

In particular, the intermediate joint may advantageously include the following ranges of motion:

second roll: -180° to 180° with respect to the second roll rotation axis, and/or
second pitch: -8° to +90° with respect to the second pitch rotation axis.

In particular, the distal joint may advantageously include the following ranges of motion:

third roll: +-inf, and/or

third pitch: -185° to 185° with respect to the third pitch rotation axis.

The first roll, the first pitch, the second roll, the second pitch and the third pitch may include driven motor stacks provided with brakes.

For instance, the first roll, the first pitch, the second roll, the second pitch and the third pitch may be actuated by frameless direct drive motors.

The third roll may include a servo motor.

Advantageously, a button can be provided for simultaneous disengagement of the motor brakes.

Accordingly, the operator is enabled to conveniently trigger motor brakes disengagement.

In particular, the button can be placed at the tip of the robot arm, so as to be easier to operate by the operator.

Preferably, the button is placed on top of the third roll.

In order to reduce temperature build-up, some components of the robot arm may be configured to receive a reduced voltage.

In particular, the second roll and the third pitch may be configured to receive a reduced voltage of 24V.

In particular, the third roll may be configured to receive an even more reduced voltage of 12V.

Lowering the applied voltage in some parts of the robot arm allows improving safety conditions.

Accordingly, patient protection measures can be mitigated.

Additionally, the efficiency in the electronics of the robot arm can be improved because there is a smaller difference between the applied and the supplied voltage A voltage of 48V is applied to the remaining parts of the robot arm.

Advantageously, the second roll, the second pitch, the third roll and the third pitch are provided with a respective cover.

The cover can be made of an electrically insulating material.

This provides a further protection.

Additionally or alternatively, the cover can be made of a thermally insulating material. Accordingly, it is easier to comply with the temperature requirements during surgery.

The cover of the second roll and the cover of the third pitch can be removable for cleaning purposes.

The remaining covers are not removable.

The present invention further provides a surgical, microsurgical or super-microsurgical robot including the robot arm as described above.

In particular, the robot may be configured and adapted to perform anastomoses.

Further details and advantages of the present invention shall now be disclosed in connection with the drawings.

It is shown in

**Fig. 1** a partial perspective view of a surgical robotic system including two robot arms;

**Fig. 2** a perspective view of a robot arm for use in surgery, microsurgery or super-microsurgery procedures, according to an embodiment of the present invention;

**Fig. 3** a perspective view similar to **Fig. 2** but from a different perspective and further showing the rotational direction of different elements of the robot arm, and

**Fig. 4** a perspective, enlarged view of a detail of covers used in the robot arm of **Fig. 2**.

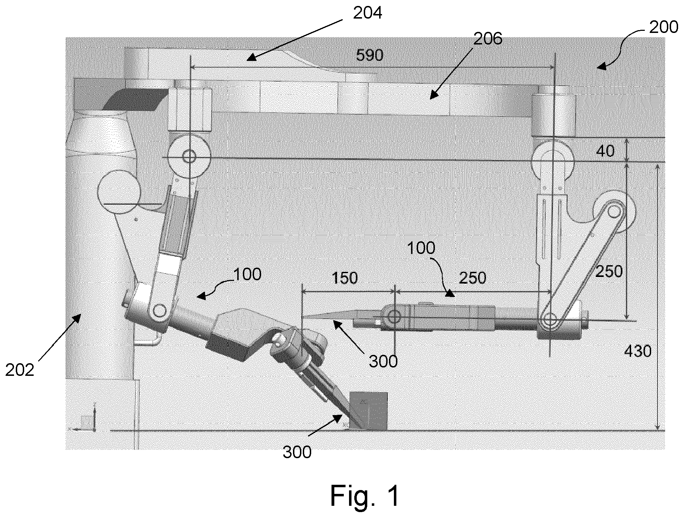

**Fig. 1** shows a partial view of a surgical robotic system 200 for surgical, microsurgical or supermicrosurgical operations.

Not shown is that the surgical robotic system 200 includes a base station.

Not shown is that the base station may include a display means for displaying information to one or more operators or provide a user interface for inserting a user input.

Not shown is that the base station may be provided with wheels allowing for easy placement of the surgical robotic system 200 at a desired location within the operating room.

The surgical robotic system 200 includes a base column 202.

The base column 202 carries a suspension arm 204, which is able to rotate about the longitudinal (perpendicular) axis of the base column 202.

The suspension arm 204 carries a fork element 206, which is able to rotate around a vertical axis mounted on the suspension arm 204.

In the shown configuration, the fork element 206 carries a first and second robot arms, such as the robot arm 100 described below.

Each of the robot arms carries a respective surgical instrument 300.

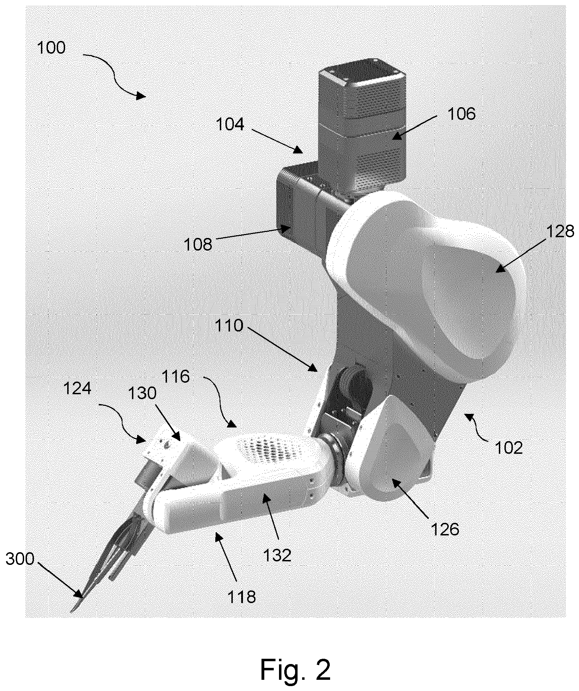

**Fig. 2** illustrates a robot arm 100 for use in surgery, microsurgery or supermicrosurgery according to an embodiment of the present invention.

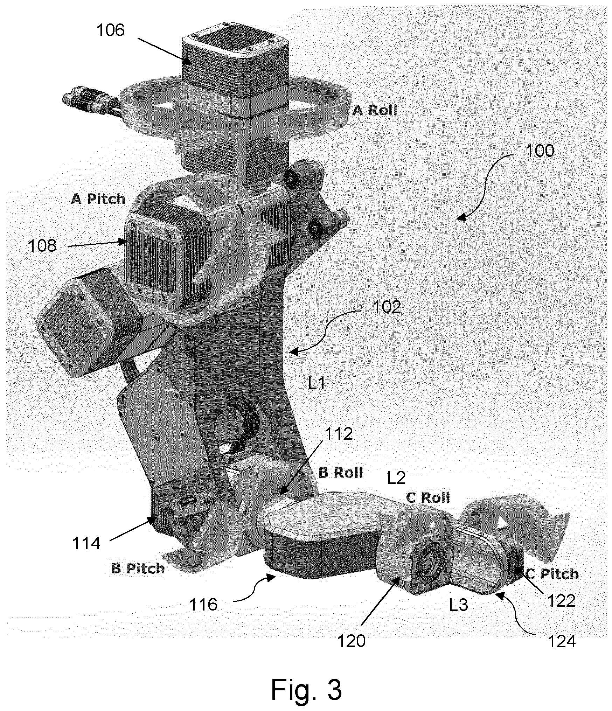

The robot arm 100 includes a first, proximal arm element 102 having a first arm element length L1 **(Fig. 3).**

A proximal end of the first arm element 102 is connected to a first, proximal joint 104 having a first roll 106 and a first pitch 108 **(Figs. 2-3).**

Also, a distal end of the first arm element 102 is connected to a second, intermediate joint 110 having a second roll 112 and a second pitch 114 **(Figs. 2-3).**

Further, the robot arm 100 includes a second, intermediate arm element 116 having a second arm element length L2 **(Fig. 3).**

A proximal end of the second arm element116 is connected to the intermediate joint 110 **(Fig. 2).**

Also, a distal end of the second arm element 116 is connected to a third, distal joint 118 having a third roll 120 and a third pitch 122 **(Figs. 2-3)**.

Still further, the robot arm 100 includes a third, distal arm element 124 having a third arm element length L3 **(Fig. 3)**.

A proximal end of the third arm element 124 is connected to the distal joint 118 **(Fig. 2)**.

In the present embodiment, the ratios between the first to second to third arm elements lengths L1, L2, L3 are:

$$(1.00 +- 10\%) : \{(1.00 \text{ to } 1.05) +- 10\%\} : (0.60 +- 10\%).$$

Preferably, the ratios between the first to second to third arm elements lengths L1, L2, L3 are:

$$(1.00 +- 3\% \text{ to } 5\%) : \{(1.00 \text{ to } 1.05) +- 3 \text{ to } 5\%\} : (0.60 +- 3\% \text{ to } 5\%).$$

In the present embodiment, the first arm element length L1 is between 225 and 275 mm.

Preferably, the first arm element length L1 is 250 mm.

In the present embodiment, the second arm element length L2 is between 231,75 and 283,25 mm.

Preferably, the second arm element length L2 is 257.5 mm.

In the present embodiment, the third arm element length L3 is between 135 and 165 mm.

Preferably, the third arm element length L3 is 150 mm.

In the present embodiment the robot arm 100 is configured to have six degrees of freedom (DOF) at its tip.

Preferably, said six degrees of freedom include three translational degrees of freedom and three rotational degrees of freedom.

This allows to perform anastomoses on microscopic level.

In the present embodiment, the proximal joint 104 includes the following ranges of motion:

first roll 106: -41° to +41° with respect to the first roll rotation axis, and/or

first pitch 108: -23° to +44° with respect to the first pitch rotation axis.

Additionally or alternatively, the intermediate joint 110 includes the following ranges of motion:

second roll 112: -180° to 180° with respect to the second roll rotation axis, and/or

second pitch 114: -8° to +90° with respect to the second pitch rotation axis.

Additionally or alternatively, the distal joint 118 includes the following ranges of motion:

third roll 120: +-inf, and/or

third pitch 122: -185° to 185° with respect to the third pitch rotation axis.

The robot arm 100 allows to obtain a high level of dexterity that is required to perform anastomoses, at the same time avoiding the risk of undesired collision with the patient during surgery.

In the present embodiment, the first roll 106, the first pitch 108, the second roll 112, the second pitch 114 and the third pitch 122 include driven motor stacks provided with brakes.

Advantageously, backlash free brakes such as permanent magnet brakes can be used.

Further, in order to reduce power consumption by the brakes, use can be made of the hysteresis in the engagement and disengagement of the brake.

The third roll 120 includes a servo motor.

In the present embodiment, a button (not shown) is provided for simultaneous disengagement of the motor brakes.

Preferably, the button is placed at the tip of the arm 100.

More preferably, the button is placed on top of the third roll 120.

Generally, the applied voltage over the elements of the robot arm 100 is 48V.

Nevertheless, in order to reduce temperature build-up, the voltage is reduced in some of the elements of the robot arm 100.

In particular, according to the present embodiment, the second roll 112 and the third pitch 122 are configured to receive a voltage of 24V.

Further, the third roll 120 is configured to receive a voltage of 12V.

Lowering the applied voltages increases the overall safety conditions of the robot arm 100, thereby improving patient protection.

In the present embodiment, covers 126, 128, 130, 132 may be provided to protect components of the robot arm 100.

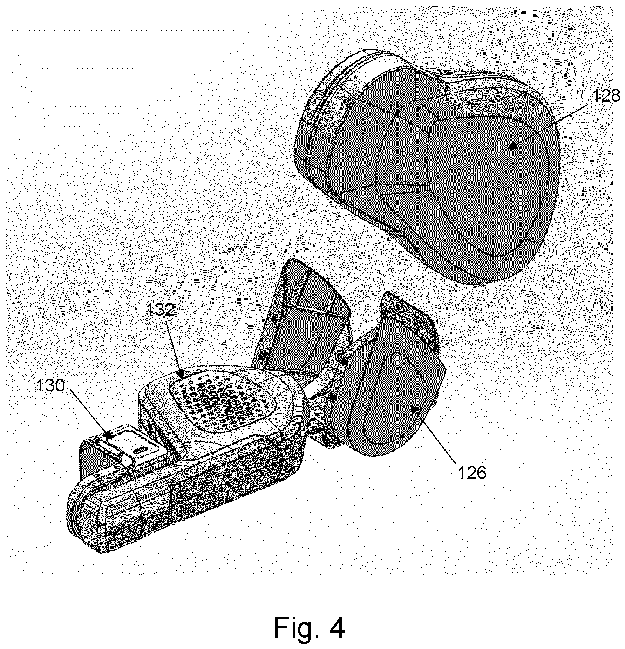

**Fig. 4** shows a detail of the covers 126, 128, 130, 132 according to the present embodiment.

In particular, a first cover 126 covers second roll 112.

A second cover 128 is provided on the intermediate arm 110 and covers the second pitch 114.

A third cover 130 covers the third roll 120.

Finally, a fourth cover 132 covers the third pitch 122.

The covers 126, 128, 130, 132 are made of an electrically insulating material and/or thermally insulating material.

Advantageously, the first and fourth covers 126, 132, respectively covering the second roll 112 and the third pitch 122 are removable for cleaning purposes.

The remaining covers 128, 130 shall not be removed.

The cover 130 of third roll 120 may have a PCB mounted on it (not shown).

A spring system (not shown) may be provided for the first pitch 108 and the second pitch 114 to compensate for torque due to. Spring position and stiffness are tuned such that the torque during surgery (e.g., anastomosis) is maintained sufficiently low, such that the motors do not excessively heat up.

The robot arm 100 can be easily draped upon being brought in an appropriate draping position.

**Reference list**

| | |
|---|---|
| 100 | Robot arm |
| 102 | Proximal arm element |
| 104 | Proximal joint |
| 106 | First roll |
| 108 | First pitch |
| 110 | Intermediate joint |
| 112 | Second roll |
| 114 | Second pitch |
| 116 | Intermediate arm element |
| 118 | Distal joint |
| 120 | Third roll |
| 122 | Third pitch |
| 124 | Distal arm element |
| 126 | Cover (of the second pitch) |
| 128 | Cover (of the second roll) |
| 130 | Cover (of the third pitch) |
| 132 | Cover (of the third roll) |
| | |
| 200 | Surgical robot system |
| 202 | Base column |
| 204 | Suspension arm |
| 206 | Fork element |
| | |
| 300 | Surgical instrument |
| | |
| L1 | First arm element length |
| L2 | Second arm element length |
| L3 | Third arm element length |

**Claims**

1.  A robot arm (100) for use in surgery, microsurgery or super-microsurgery, said robot arm (100) comprising:

    a first, proximal arm element (102) having a first arm length (L1), the proximal arm element (102) being connected to a first, proximal joint (104) having a first roll (106) and a first pitch (108), and a second, intermediate joint (110) having a second roll (112) and a second pitch (114);
    a second, intermediate arm element (116) having a second arm length (L2), the intermediate arm element (116) being connected to the intermediate joint (110) and a third, distal joint (118) having a third roll (120) and a third pitch (122), and
    a third, distal arm element (124) having a third arm length (L3), the third arm element (124) being connected to the distal joint (118),
    wherein the ratios between the first to second to third arm element lengths (L1, L2, L3) are:

    $$(1.00 +- 10\%) :\{ (1.00 \text{ to } 1.05) +- 10\%\} : (0.60 +- 10\%).$$

2.  The robot arm (100) according to claim 1,
    **characterized in that**
    the ratio between the first to third arm element lengths (L1, L2, L3) is:

    $$(1.00 +- 3\% \text{ to } 5\%) : \{(1.00 \text{ to } 1.05) +- 3 \text{ to } 5\%\} : (0.60 +- 3\% \text{ to } 5\%).$$

3.  The robot arm (100) according to claim 1,
    **characterized in that:**

    - the first arm element length (L1) is between 225 and 275 mm;
    - the second arm element length (L2) is between 231,75 and 283,25 mm;
    - the third arm element length (L3) is between 135and 165 mm.

4.  The robot arm (100) according to claim 4,
    **characterized in that** :

    - the first arm element length (L1) is 250 mm;
    - the second arm element length (L2) is 257.5 mm;
    - the third arm element length (L3) is 150 mm.

5.  The robot arm (100) according to any one of the preceding claims,
    **characterized in that**
    the robot arm (100) is configured to have six degrees of freedom at its tip, preferably wherein said six degrees of freedom include three translational degrees of freedom and three rotational degrees of freedom.

6.  The robot arm (100) according to any one of the preceding claims,
    **characterized in that**
    the proximal joint (104) includes the following ranges of motion:

    first roll (106): -41° to +41° with respect to the first roll rotation axis, and/or
    first pitch (108): -23° to +44° with respect to the first pitch rotation axis.

7.  The robot arm (100) according to any one of the preceding claims,
    **characterized in that**
    the intermediate joint (110) includes the following ranges of motion:

second roll (112): -180° to 180° with respect to the second roll rotation axis, and/or
second pitch (114): -8° to +90° with respect to the second pitch rotation axis.

8.  The robot arm (100) according to any one of the preceding claims,
    **characterized in that**
    the distal joint (118) includes the following ranges of motion:

    - third roll (120): +-inf, and/or
    - third pitch (122): -185° to 185° with respect to the third pitch rotation axis.

9.  The robot arm (100) according to any one of the preceding claims,
    **characterized in that:**

    the first roll (106), the first pitch (108), the second roll (112), the second pitch (114) and the third pitch (122)
    include driven motor stacks provided with brakes, and
    the third roll (120) includes a servo motor.

10. The robot arm (100) according to claim 9,
    **characterized in that**
    a button is provided for simultaneous disengagement of the motor brakes.

11. The robot arm (100) according to claim 10,
    **characterized in that**
    said button is placed at the tip of the arm (100), preferably wherein said button is placed on top of the third roll (120).

12. The robot arm (100) according to any one of the preceding claims,
    **characterized in that**

    the second roll (112) and the third pitch (122) are configured to receive a voltage of 24V,
    the third roll (120) is configured to receive a voltage of 12V, and
    the remaining parts of the robot arm (100) are configured to receive a voltage of 48V.

13. The robot arm (100) according to any one of the preceding claims,
    **characterized in that**
    the second roll (112), the second pitch (114), the third roll (120) and the third pitch (122) are provided with a respective
    cover (126, 128, 130, 132) made of an electrically and/or thermally insulating material.

14. The robot arm (100) according to claim 13,
    **characterized in that**
    the cover (126) of the second roll (112) and the cover (132) of the third pitch (122) are removable.

15. A surgical, microsurgical or super-microsurgical robot (200), in particular for performing anastomoses including the
    robot arm (100) according to any one of the preceding claims.

Fig. 1

Fig. 2

Fig. 3

Fig. 4

**EUROPEAN SEARCH REPORT**

Europäisches Patentamt
European Patent Office
Office européen des brevets

**Application Number**

EP 22 16 9326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 134 006 B1 (BIO MEDICAL ENG HK LTD [HK]) 12 February 2020 (2020-02-12) | 1,2,5-9, 12-15 | INV. A61B34/30 |
| A | * paragraphs [0028], [0058], [0067], [0076], [0081]; figures 1, 7a-b, 8a * | 3,4,10, 11 | A61B34/00 B25J7/00 B25J17/02 |
| X | WO 2016/181164 A1 (CAMBRIDGE MEDICAL ROBOTICS LTD [GB]) 17 November 2016 (2016-11-17) | 1,2,5-9, 12-15 | |
| Y | * page 5, line 5 – page 9, line 29 * | 10,11 | |
| A | * page 12, lines 11-21 * <br> * figures 3, 4a * | 3,4 | |
| A | US 6 233 504 B1 (DAS HARI [US] ET AL) 15 May 2001 (2001-05-15) * column 4, lines 29-56; figure 2 * | 1-15 | |
| A | WO 2018/053349 A1 (VERB SURGICAL INC [US]) 22 March 2018 (2018-03-22) * the whole document * | 1-15 | |
| A | WANG LIANDONG ET AL: "The design of a dual channel synchronous control system based on a new percutaneous puncture surgical robot", MULTIMEDIA TOOLS AND APPLICATIONS, KLUWER ACADEMIC PUBLISHERS, BOSTON, US, vol. 79, no. 15-16, 18 July 2019 (2019-07-18), pages 10405-10425, XP037110130, ISSN: 1380-7501, DOI: 10.1007/S11042-019-07891-9 [retrieved on 2019-07-18] * particularly fig. 4; the whole document * | 1-15 | TECHNICAL FIELDS SEARCHED (IPC) <br> A61B <br> B25J |

–/–

The present search report has been drawn up for all claims

| Place of search | Date of completion of the search | Examiner |
|---|---|---|
| The Hague | 3 October 2022 | Rosander, Frida |

Europäisches
Patentamt

European
Patent Office

Office européen
des brevets

# EUROPEAN SEARCH REPORT

**Application Number**

EP 22 16 9326

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HUANG ZHIFENG ET AL: "Motion Planning for Bandaging Task With Abnormal Posture Detection and Avoidance", IEEE/ASME TRANSACTIONS ON MECHATRONICS, IEEE SERVICE CENTER, PISCATAWAY, NJ, US, vol. 25, no. 5, 12 February 2020 (2020-02-12), pages 2364-2375, XP011814781, ISSN: 1083-4435, DOI: 10.1109/TMECH.2020.2973674 [retrieved on 2020-10-14] * particularly fig. 3; the whole document * | 1-15 | |
| Y | EP 3 977 957 A2 (MEDICAL MICROINSTRUMENTS SPA [IT]) 6 April 2022 (2022-04-06) * paragraphs [0188], [0204], [0206], [0603]; figure 8 * | 10,11 | |

**TECHNICAL FIELDS SEARCHED (IPC)**

The present search report has been drawn up for all claims

| Place of search | Date of completion of the search | Examiner |
|---|---|---|
| The Hague | 3 October 2022 | Rosander, Frida |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT
### ON EUROPEAN PATENT APPLICATION NO.

EP 22 16 9326

This annex lists the patent family members relating to the patent documents cited in the above-mentioned European search report.
The members are as contained in the European Patent Office EDP file on
The European Patent Office is in no way liable for these particulars which are merely given for the purpose of information.

03-10-2022

| Patent document cited in search report | | Publication date | Patent family member(s) | | Publication date |
|---|---|---|---|---|---|
| EP 3134006 | B1 | 12-02-2020 | CN | 105358072 A | 24-02-2016 |
| | | | CN | 108836480 A | 20-11-2018 |
| | | | CN | 109907828 A | 21-06-2019 |
| | | | EP | 3134006 A1 | 01-03-2017 |
| | | | HK | 1219402 A1 | 07-04-2017 |
| | | | US | 2015297299 A1 | 22-10-2015 |
| | | | US | 2017128143 A1 | 11-05-2017 |
| | | | US | 2019000573 A1 | 03-01-2019 |
| | | | US | 2022022734 A1 | 27-01-2022 |
| | | | WO | 2015161677 A1 | 29-10-2015 |
| WO 2016181164 | A1 | 17-11-2016 | CN | 107666875 A | 06-02-2018 |
| | | | CN | 112137724 A | 29-12-2020 |
| | | | EP | 3294183 A1 | 21-03-2018 |
| | | | GB | 2538497 A | 23-11-2016 |
| | | | JP | 6678686 B2 | 08-04-2020 |
| | | | JP | 2018516656 A | 28-06-2018 |
| | | | US | 2016331482 A1 | 17-11-2016 |
| | | | US | 2019110850 A1 | 18-04-2019 |
| | | | US | 2021298858 A1 | 30-09-2021 |
| | | | WO | 2016181164 A1 | 17-11-2016 |
| US 6233504 | B1 | 15-05-2001 | US | 6233504 B1 | 15-05-2001 |
| | | | US | 2001020200 A1 | 06-09-2001 |
| WO 2018053349 | A1 | 22-03-2018 | AU | 2017326462 A1 | 07-03-2019 |
| | | | AU | 2020203372 A1 | 11-06-2020 |
| | | | BR | 112019004223 A2 | 28-05-2019 |
| | | | CA | 3034639 A1 | 22-03-2018 |
| | | | CN | 108472028 A | 31-08-2018 |
| | | | EP | 3512436 A1 | 24-07-2019 |
| | | | JP | 6783925 B2 | 11-11-2020 |
| | | | JP | 2019534060 A | 28-11-2019 |
| | | | KR | 20190049728 A | 09-05-2019 |
| | | | US | 2018079090 A1 | 22-03-2018 |
| | | | US | 2020306997 A1 | 01-10-2020 |
| | | | WO | 2018053349 A1 | 22-03-2018 |
| EP 3977957 | A2 | 06-04-2022 | AU | 2016337026 A1 | 10-05-2018 |
| | | | AU | 2016337029 A1 | 10-05-2018 |
| | | | AU | 2021250974 A1 | 11-11-2021 |
| | | | BR | 112018007606 A2 | 23-10-2018 |
| | | | BR | 112018007640 A2 | 06-11-2018 |
| | | | CA | 3001934 A1 | 20-04-2017 |
| | | | CA | 3001935 A1 | 20-04-2017 |
| | | | CN | 108366837 A | 03-08-2018 |

EPO FORM P0459

For more details about this annex : see Official Journal of the European Patent Office, No. 12/82

**page 1 of 2**

EP 4 265 216 A1

## ANNEX TO THE EUROPEAN SEARCH REPORT
## ON EUROPEAN PATENT APPLICATION NO.

EP 22 16 9326

03-10-2022

| Patent document cited in search report | Publication date | Patent family member(s) | | Publication date |
|---|---|---|---|---|
| | | CN | 108366838 A | 03-08-2018 |
| | | CN | 113768629 A | 10-12-2021 |
| | | EP | 3361980 A1 | 22-08-2018 |
| | | EP | 3361981 A1 | 22-08-2018 |
| | | EP | 3977957 A2 | 06-04-2022 |
| | | JP | 6865970 B2 | 28-04-2021 |
| | | JP | 6942360 B2 | 29-09-2021 |
| | | JP | 2018534099 A | 22-11-2018 |
| | | JP | 2018534100 A | 22-11-2018 |
| | | JP | 2021106894 A | 29-07-2021 |
| | | JP | 2022000161 A | 04-01-2022 |
| | | KR | 20180073608 A | 02-07-2018 |
| | | KR | 20180074712 A | 03-07-2018 |
| | | KR | 20210152594 A | 15-12-2021 |
| | | US | 2018296285 A1 | 18-10-2018 |
| | | US | 2018303567 A1 | 25-10-2018 |
| | | US | 2021059776 A1 | 04-03-2021 |
| | | US | 2021386495 A1 | 16-12-2021 |
| | | US | 2021386496 A1 | 16-12-2021 |
| | | WO | 2017064301 A1 | 20-04-2017 |
| | | WO | 2017064303 A1 | 20-04-2017 |

EPO FORM P0459

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2731535 A1